# EUROPÄISCHE PATENTSCHRIFT

(11) **EP 3 082 894 B1**
(45) Veröffentlichungstag und Bekanntmachung des Hinweises auf die Patenterteilung: **28.02.2018**
(21) Anmeldenummer: 14821084.2
(22) Anmeldetag: 08.12.2014
(51) Int. Cl.: A61M 1/00

(54) **SYSTEM ZUR KOMBINIERTEN UNTERDRUCK- UND INSTILLATIONSBEHANDLUNG VON WUNDEN**
SYSTEM FOR COMBINED VACUUM AND INSTILLATION TREATMENT OF WOUNDS
SYSTÈME DE TRAITEMENT COMBINÉ DE BLESSURES PAR DÉPRESSION ET INSTILLATION

(30) Priorität: 19.12.2013 DE 102013226713
(43) Veröffentlichungstag der Anmeldung: 26.10.2016
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); BEYRLE, Karina, 89075 Ulm (DE)
(74) Vertreter: DREISS Patentanwälte PartG mbB
(86) Internationale Anmeldenummer: PCT/EP2014/076924
(87) Internationale Veröffentlichungsnummer: WO 2015/091072

(56) Entgegenhaltungen:
- DE-A1- 4 219 890
- US-A- 4 136 696
- US-A- 6 013 048
- US-A1- 2007 233 003
- US-A1- 2012 289 894

## Beschreibung

Die Erfindung betrifft ein System zur kombinierten Unterdruck- und Instillationsbehandlung von Wunden am menschlichen oder tierischen Körper, wobei das System umfasst, eine Unterdruckeinheit mit einem Gehäuse einer ersten Gehäuseart, mit einer Pumpe zur Bereitstellung von Unterdruck und mit einem Flüssigkeitsaufnahmebehälter zur Aufnahme von Körperflüssigkeiten, insbesondere von aus einer Wunde abgesaugten Wundsekreten, wobei der Behälter lösbar an dem Gehäuse der ersten Gehäuseart befestigbar ist, und eine Instillationseinheit mit einem Gehäuse einer zweiten Gehäuseart mit einem Leitungsdurchführungsbereich für mindestens einen eingehenden Leitungsabschnitt und einen ausgehenden Leitungsabschnitt, mit einer Förderpumpe, vorzugsweise einer Peristaltikpumpe, wobei die Gehäuse der ersten und der zweiten Gehäuseart jeweils separate voneinander getrennte oder voneinander trennbare Gehäuse sind.

Unterdrucktherapie wie auch Instillationstherapie finden beispielsweise bei der Behandlung von schwer heilenden Wunden Anwendung. Bei der Behandlung von Wunden mit Unterdruck wird eine druckdichte Abdeckung über der Wunde angebracht und der Raum zwischen Abdeckung und Wunde wird mit Unterdruck beaufschlagt. Dies fördert die Wundheilung. Instillationstherapie bezeichnet eine Behandlung von Wunden, bei der fluide Medien, insbesondere flüssige Arzneimittel in die Wunde eingebracht werden. Beispiele für solche flüssigen Medien sind Lösungen, Emulsionen oder Suspensionen von Antibiotika, Schmerzmitteln oder Lokalanästhetika. Hierdurch kann beispielsweise eine die Wundheilung beeinträchtigende Entzündung im Wundbereich behandelt oder verhindert werden (siehe z.B. US6013048).

Die kombinierte Anwendung von Unterdruck- und Instillationsbehandlung vereint die Vorteile der beiden Behandlungsmethoden. Unter separaten voneinander getrennten oder trennbaren Gehäusen sind im Sinne der vorliegenden Erfindung Gehäuse zu verstehen, welche eine separate Handhabung der Instillationseinheit bzw. der Unterdruckeinheit erlauben. Hierbei sind die separaten Gehäuse derart ausgeführt, dass die Instillationseinheit bzw. die Unterdruckeinheit funktional getrennte Einheiten darstellen, das heißt, dass keine Komponenten der Instillationseinheit im Gehäuse der Unterdruckeinheit untergebracht sind und umgekehrt keine Komponenten der Unterdruckeinheit im Gehäuse der Instillationseinheit untergebracht sind. Hiervon ausgenommen sind Steuerungskomponenten im Sinne einer Master-Slave Steuerung.

Aufgabe der vorliegenden Erfindung ist es, ein System zu schaffen, welches die Behandlung von Wunden mittels kombinierter Instillations- und Unterdrucktherapie vereinfacht, insbesondere die Bedienung und Handhabung der dazu verwendeten Geräte verbessert.

Diese Aufgabe wird bei einem System der eingangs genannten Art erfindungsgemäß dadurch gelöst, dass das Gehäuse der zweiten Gehäuseart eine öffenbare und verschließbare Abdeckung aufweist, über die der Leitungsdurchführungsbereich zugänglich ist, und dass das Gehäuse der ersten Gehäuseart eine erste Befestigungseinrichtung aufweist und dass das Gehäuse der zweiten Gehäuseart eine zweite Befestigungseinrichtung aufweist, wobei die erste Befestigungseinrichtung mit der zweiten Befestigungseinrichtung formschlüssig, vorzugsweise schnappend, rastend oder in sonstiger Weise formschlüssig hintergreifend, oder klemmend verbindbar ist. Hierdurch sind die Unterdruckeinheit und die Instillationseinheit zu einer handhabbaren Geräteeinheit zusammenfügbar. Besonders vorteilhaft ist hierbei eine Ausbildung bei der die beiden Einheiten zu einem Geräteturm zusammenfügbar sind. Die erfindungsgemäß ausgestaltete Abdeckung ermöglicht hierbei einen einfachen Zugriff auf den Leitungsdurchführungsbereich, in welchem die Leitungen der Instillationseinheit angeordnet bzw. anordenbar sind. Es erweist sich als vorteilhaft, wenn die Abdeckung schwenkbar an dem Gehäuse der zweiten Gehäuseart angebracht ist. Hierdurch kann sich die Abdeckung nicht unbeabsichtigt von dem Gehäuse der zweiten Gehäuseart lösen; eine einfache Zugänglichkeit des Leitungsdurchführungsbereichs ist jedoch weiterhin gewährleistet.

Im Sinne der Erfindung ist dabei ebenso, wenn die Abdeckung bezüglich einer Betriebsposition des Systems seitlich oder hinten oder vorn an dem Gehäuse der zweiten Gehäuseart angebracht ist. Eine solche Anbringung der Abdeckung ist besonders vorteilhaft, wenn die Instillationseinheit und die Unterdruckeinheit zu einem Geräteturm zusammenfügt sind. In diesem Fall gewährleistet eine Anbringung der Abdeckung seitlich, hinten oder vorn weiterhin die Möglichkeit die Abdeckung zu öffnen, ohne die Instillationseinheit und die Unterdruckeinheit voneinander lösen zu müssen.

Im Sinne der Erfindung ist ebenso, wenn die Abdeckung den Leitungsdurchführungsbereich der Instillationseinheit begrenzt. Das Innenleben des Leitungsdurchführungsbereichs wird also durch die Abdeckung vor äußeren Einflüssen geschützt. Durch die Möglichkeit, die Abdeckung zu öffnen, bleibt der Leitungsdurchführungsbereich zugänglich.

Eine Ausführungsform des erfindungsgemäßen Systems zeichnet sich dadurch aus, dass bezüglich einer Betriebsposition des Systems die erste Befestigungseinrichtung an einer Unterseite des Gehäuses der ersten Gehäuseart angeordnet ist und die zweite Befestigungseinrichtung an einer Oberseite des Gehäuses der zweiten Gehäuseart angeordnet ist. Hierdurch können die Unterdruck- und die Instillationseinheit übereinander angeordnet und betriebssicher miteinander verbunden werden, wodurch ihre Handhabbarkeit erhöht wird.

Nach einer weiteren Ausführungsform der Erfindung weist das Gehäuse der zweiten Gehäuseart mindestens eine dritte Befestigungseinrichtung auf, wobei diese dritte Befestigungseinrichtung mit einer zweiten Befestigungseinrichtung eines weiteren Gehäuses der zweiten Gehäuseart formschlüssig, vorzugsweise schnappend, rastend oder in sonstiger Weise formschlüssig hintergreifend, oder klemmend verbindbar ist, und wobei die dritte Befestigungseinrichtung bezüglich einer Betriebsposition des Systems vorzugsweise an einer Unterseite des Gehäuses der zweiten Gehäuseart angeordnet ist. Dies ermöglicht es, unterhalb einer Unterdruckeinheit mehrere Instillationseinheiten anzuordnen. Hierdurch kann eine oder mehrere zu behandelnde Wunden beispielsweise mit mehreren Instillationsflüssigkeiten behandelt werden, wobei die verschiedenen Instillationsflüssigkeiten von verschiedenen Instillationseinheiten gefördert werden, wodurch beispielsweise unterschiedliche Förderraten sowie unterschiedliche Zusammensetzung der Instillationsflüssigkeiten ermöglicht werden.

Im Sinne der Erfindung ist ebenso ein System, welches eine Haltevorrichtung mit einer der Haltevorrichtung zugeordneten Befestigungseinrichtung umfasst, wobei diese der Haltevorrichtung zugeordnete Befestigungseinrichtung mit der ersten Befestigungseinrichtung des Gehäuses der ersten Art und/oder der dritten Befestigungseinrichtung des Gehäuses der zweiten Gehäuseart formschlüssig, vorzugsweise schnappend, rastend oder in sonstiger Weise formschlüssig hintergreifend, oder klemmend verbindbar ist. Hierdurch kann entweder die Unterdruckeinheit direkt mit der Haltevorrichtung verbunden werden oder eine Instillationseinheit an der Haltevorrichtung befestigt werden und gegebenenfalls weitere Instillationseinheiten an der mit der Haltevorrichtung verbundenen Instillationseinheit befestigt werden. Oberhalb der Instillationseinheit oder der Instillationseinheiten könnte eine Unterdruckeinheit angeordnet werden. Auf diese Weise kann quasi ein Turm von Instillationseinheiten und einer Unterdruckeinheit mittels der Haltevorrichtung gehalten oder gestützt werden. Über die Haltevorrichtung kann dieser Turm dann beispielsweise mittels einer an der Haltevorrichtung angeordneten Klemmvorrichtung an beispielsweise einem Infusionsständer oder einem Krankenbett befestigt werden.

Besonders vorteilhaft ist hierbei, wenn die Instillationseinheit oder die Unterdruckeinheit eine Schnittstelle zur Spannungsversorgung aufweist, welche mit einer komplementären Schnittstelle zur Spannungsversorgung bei der Haltevorrichtung verbindbar ist. Vorzugsweise sind dabei die zueinander komplementären Schnittstellen zur Spannungsversorgung derart ausgestaltet, dass bei einer Verbindung der Instillationseinheit mit der Haltevorrichtung ein Kontakt zwischen den beiden Schnittstellen zustande kommt, sodass eine Spannungsversorgung der Instillationseinheit durch eine Befestigung der Instillationseinheit an der Haltevorrichtung gewährleistet ist.

Eine Fortbildung des erfindungsgemäßen Systems zeichnet sich dadurch aus, dass die Instillationseinheit eine Schnittstelle zur Spannungsversorgung aufweist, welche mit einer komplementären Schnittstelle zur Spannungsversorgung bei der Unterdruckeinheit verbindbar ist. Hierdurch können die Unterdruckeinheit und eine bzw. mehrere Instillationseinheiten an der Haltevorrichtung befestigt werden und eine Spannungsversorgung zwischen den einzelnen Einheiten wird über die Schnittstellen zur Spannungsversorgung gewährleistet, ohne dass Kabel zur Stromversorgung bzw. Spannungsversorgung eingesetzt werden müssen. Vorteilhafterweise sind die Schnittstellen dabei derart ausgebildet, dass beim Verbinden der einzelnen Einheiten miteinander ein Kontakt zur Spannungsversorgung automatisch zustande kommt. Beispielsweise können die Instillationseinheit und die Unterdruckeinheit mittels schiebesitzartiger Verbindungselemente aufeinander aufschiebbar ausgebildet sein, wobei die zueinander komplementären Schnittstellen in diesem Fall derart ausgebildet sind, dass beim Aufeinanderschieben der Instillations- und Unterdruckeinheit ein Kontakt zwischen den Schnittstellen zur Spannungsversorgung entsteht. Gleiches gilt für die Ausbildung der Schnittstelle bei der Haltevorrichtung bzw. den zu dieser Schnittstelle komplementären Schnittstellen der Unterdruckeinheit bzw. der Instillationseinheit.

Erfindungsgemäß ist ebenso ein System, bei welchem der Leitungsdurchführungsbereich für mindestens zwei eingehende Leitungsabschnitte und mindestens einen ausgehenden Leitungsabschnitt ausgelegt ist. Hierdurch können eingehende Leitungsabschnitte mit verschiedenen Instillationsflüssigkeiten verbunden werden und diese dann alternativ oder gemeinsam dem ausgehenden Leitungsabschnitt zugeführt werden.

In einer Weiterbildung des eben beschriebenen Systems werden die eingehenden Leitungsabschnitte in dem Leitungsdurchführungsbereich zusammengeführt und münden in den ausgehenden Leitungsabschnitt. Hierdurch wird es möglich, mittels einer Instillationseinheit mehrere Instillationsflüssigkeiten zu kombinieren bzw. gemischt einer Wunde zuzuführen. Hierdurch kann beispielsweise die Anwendung einer Antibiotikalösung mit der Anwendung eines Schmerzmittels kombiniert werden. Vorzugsweise sind im Bereich des Leitungsdurchführungsbereichs bei dieser Ausführungsform Durchflusssteuer- oder -regelelemente angeordnet, über welche die Fluidförderraten in den eingehenden Leitungsabschnitten gesteuert oder geregelt werden können. Solche Durchflusssteuer- oder -regelelemente können beispielsweise Ventile oder auch Klemmen sein.

Vorteilhaft ist außerdem, wenn die Förderpumpe, welche vorzugsweise als Peristaltikpumpe ausgestaltet ist, derart im Bereich des ausgehenden Leitungsabschnitts angeordnet und ausgebildet ist, dass sie im Betrieb fluidfördernd auf den ausgehenden Leitungsabschnitt einwirkt. Das Einwirken der Förderpumpe auf den ausgehenden Leitungsabschnitt ermöglicht es, mehrere eingehende Leitungsabschnitte mit lediglich einer Förderpumpe zu betreiben. Hierdurch kann die Größe der Instillationseinheit des Systems reduziert werden bzw. Kosten für den Einbau einer zusätzlichen Förderpumpe gespart werden.

Im Sinne der Erfindung ist ebenso, wenn die Instillationseinheit einen Sensor aufweist, der entlang einer Fluidförderrichtung vor der Förderpumpe angeordnet ist und ausgebildet ist, um zu detektieren, wenn der eingehende Leitungsabschnitt keine Instillationsflüssigkeit enthält. Wenn die zu fördernde Instillationsflüssigkeit zuneige geht, wird dies durch den genannten Sensor detektiert, und die Förderung der Instillationsflüssigkeit kann einstellt und ein Signal ausgegeben werden, so dass zusätzliche Instillationsflüssigkeit bereitgestellt wird.

Im Sinne der Erfindung ist ebenso, wenn die Instillationseinheit eine Temperiervorrichtung umfasst, die zur Temperatursteuerung und/oder -regelung von zu fördernder Instillationsflüssigkeit ausgelegt ist. Eine solche Temperiervorrichtung kann beispielsweise durch ein Heizelement, welches im Bereich des ausgehenden oder eingehenden Leitungsabschnitts angeordnet ist, realisiert werden. Vorteilhafterweise ist im Bereich des ausgehenden Leitungsabschnitts dabei ein Temperatursensor angeordnet, welcher in Förderrichtung der Instillationsflüssigkeit nach dem Heizelement angeordnet ist. Ein solcher Sensor erlaubt die Konstruktion eines Regelkreises, sodass die Temperatur der zu fördernden Instillationsflüssigkeit genau eingestellt werden kann.

Eine Weiterbildung des erfindungsgemäßen Systems zeichnet sich dadurch aus, dass die Abdeckung ausgebildet ist, um im geschlossenen Zustand den Leitungsdurchführungsbereich zu begrenzen und einen Leitungsabschnitt zu führen und/oder zu fixieren. Dies kann beispielsweise durch eine Ausführung der Abdeckung erreicht werden, bei der die Abdeckung eine rinnenartige Ausnehmung aufweist, in welche im Leitungsdurchführungsbereich angeordnete Leitungsabschnitte beim Schließen der Abdeckung eingebracht werden. Hierdurch wird verhindert, dass die Leitungsabschnitte verrutschen oder aus dem Leitungsdurchführungsbereich herausgezogen werden.

Im Sinne der Erfindung ist ebenso, wenn im Leitungsdurchführungsbereich mindestens ein Mittel vorgesehen ist, welches angeordnet und ausgelegt ist, um einen im Leitungsdurchführungsbereich anordenbaren Leitungsabschnitt fluidisch zu unterbrechen, insbesondere abzuklemmen. Hierdurch kann beispielsweise die Förderung der Instillationsflüssigkeit aus einem eingehenden Leitungsabschnitt kurzzeitig unterbrochen werden, während aus einem anderen eingehenden Leitungsabschnitt gefördert wird. Dies erhöht die Flexibilität des erfindungsgemäßen Systems in der Anwendung.

Im Sinne der Erfindung ist ebenso ein System, wie beansprucht, welches mindestens zwei Instillationseinheiten umfasst. Hierdurch ergibt sich der Vorteil, dass zwei Instillationsflüssigkeiten unabhängig voneinander der Wunde zugeführt werden können. Ebenso können die beiden Instillationseinheiten im Wechsel zueinander betrieben werden, d.h., sobald eine der Instillationseinheiten keine Instillationsflüssigkeit mehr fördern kann, wird auf die andere Instillationseinheit umgeschaltet, so dass eine nahtlose Versorgung der Wunde mit Instillationsflüssigkeit gewährleistet ist.

Eine Weiterbildung der Erfindung zeichnet sich dadurch aus, dass die Unterdruckeinheit eine Steuerungseinheit umfasst und dass Signale der Steuerungseinheit über eine Schnittstelle, insbesondere eine Bluetooth-Schnittstelle oder eine sonstige Schnittstelle zur kabellosen Kommunikation, oder eine Schnittstelle zur kabelbasierten Kommunikation, insbesondere über eine USB-Schnittstelle, an die Instillationseinheit übermittelt werden. Hierdurch ist ein Betrieb des Systems möglich, bei welchem die Unterdruckeinheit als Master-Einheit fungiert und die Instillationseinheit als Slave-Einheit. Vorzugsweise kann die Unterdruckeinheit dabei mehrere Instillationseinheiten gleichzeitig betreiben. Die Unterdruckeinheit kann dabei eine Bedienoberfläche, beispielsweise in Form eines berührungsempfindlichen Bildschirms, aufweisen, über welches das gesamte System, d.h., die Unterdruckeinheit und die Instillationseinheit bzw. die Instillationseinheiten betrieben werden können.

Besonders vorteilhaft ist dabei, wenn die Steuerungseinheit derart ausgebildet ist, dass sie eine Instillationseinheit, welche über die Schnittstelle mit der Unterdruckeinheit verbunden ist, erkennt und den Betrieb der Unterdruckeinheit derart anpasst, dass sie einen Betrieb der Instillationseinheit beim Betrieb der Unterdruckeinheit berücksichtigt. Hiermit ist gemeint, dass die Beaufschlagung der Wunde mit Unterdruck entsprechend angepasst wird, je nachdem ob eine Instillationseinheit zur Unterdruckbehandlung parallel betrieben wird oder nicht. Bei gleichzeitiger Behandlung der Wunde mit Unterdruck und Instillationsflüssigkeit kann es vorteilhaft sein, wenn die Förderrate der Unterdruckeinheit erhöht wird, sodass die in die Wunde eingebrachte Instillationsflüssigkeit über die Unterdruckeinheit aus der Wunde abgeführt wird.

Alternativ hierzu ist es ebenso im Sinne der Erfindung, wenn die Instillationseinheit eine Steuerungseinheit umfasst und Signale der Steuerungseinheit über eine Schnittstelle, insbesondere über eine Bluetooth-Schnittstelle oder eine sonstige Schnittstelle zur kabellosen Kommunikation, oder eine Schnittstelle zur kabelbasierten Kommunikation, insbesondere über eine USB-Schnittstelle, an die Unterdruckeinheit übermittelt werden. Diese Ausführungsform stellt den umgekehrten Fall zu der eben beschriebenen Ausführungsform dar, sodass nun die Instillationseinheit als Master-Einheit des Systems und die Unterdruckeinheit als Slave-Einheit fungiert. In dieser Ausführungsform ist es ebenso möglich, dass eine Instillationseinheit sowohl die Unterdruckeinheit als auch zusätzlich eine oder mehrere weitere Instillationseinheiten steuert.

Der Betrieb des gesamten Systems mittels einer einzigen Master-Steuerungseinheit bietet den Vorteil, dass die einzelnen Einheiten abgestimmt aufeinander betrieben werden können. Es ergeben sich überdies Vorteile bezüglich der Protokollierung des Behandlungsverlaufs. So können die Betriebsparameter aller im System befindlicher Einheiten zentral aufgezeichnet werden und damit der Betriebsverlauf archiviert werden.

Im Sinne der Erfindung ist auch, dass die Steuerungseinheit derart ausgebildet ist, dass sie eine optional vorhandene Instillationseinheit beziehungsweise eine optional vorhandene Unterdruckeinheit über die Schnittstelle erkennt und dass sie im Erkennungsfall auf der Steuerungseinheit eine bislang inaktive Programmroutine startet, insbesondere eine bislang inaktive Programmroutine startet, welche eine Benutzeroberfläche von einem ersten Zustand in einen zweiten Zustand schaltet, insbesondere bislang inaktive Optionen in der Benutzeroberfläche auswählbar schaltet. Hierdurch können beispielsweise Optionen in der Benutzeroberfläche einer Unterdruckeinheit ausgeblendet oder deaktiviert bleiben, solange keine Instillationseinheit mit ihr verbunden ist und die Unterdruckeinheit allein betrieben wird. Wird eine Instillationseinheit mit der Unterdruckeinheit verbunden und durch die Steuerungseinheit erkannt, so werden in der Benutzeroberfläche der Unterdruckeinheit Optionen auswählbar geschaltet, welche vorher nicht auswählbar waren oder überhaupt nicht dargestellt wurden, beispielsweise zusätzliche Wahloptionen oder Betriebsoptionen zu den beiden Einheiten.

Weitere Merkmale, Einzelheiten und Vorteile der Erfindung ergeben sich aus den beigefügten Patentansprüchen, der zeichnerischen Darstellung der nachfolgenden Beschreibung einer bevorzugten Ausführungsform des erfindungsgemäßen Systems.

In der Zeichnung zeigen:
Figur 1: eine perspektivische Darstellung einer Ausführungsform des erfindungsgemäßen Systems,
Figur 2: eine Seitenansicht des Systems aus Figur 1,
Figur 3: eine perspektivische Darstellung eines Teils des Systems aus Figur 1,
Figur 4: eine perspektivische Darstellung einer weiteren Ausführungsform des erfindungsgemäßen Systems,
Figur 5: eine Frontansicht einer weiteren Ausführungsform des erfindungsgemäßen Systems, und
Figur 6: eine perspektivische Darstellung einer Instillationseinheit einer weiteren Ausführungsform des erfindungsgemäßen Systems.

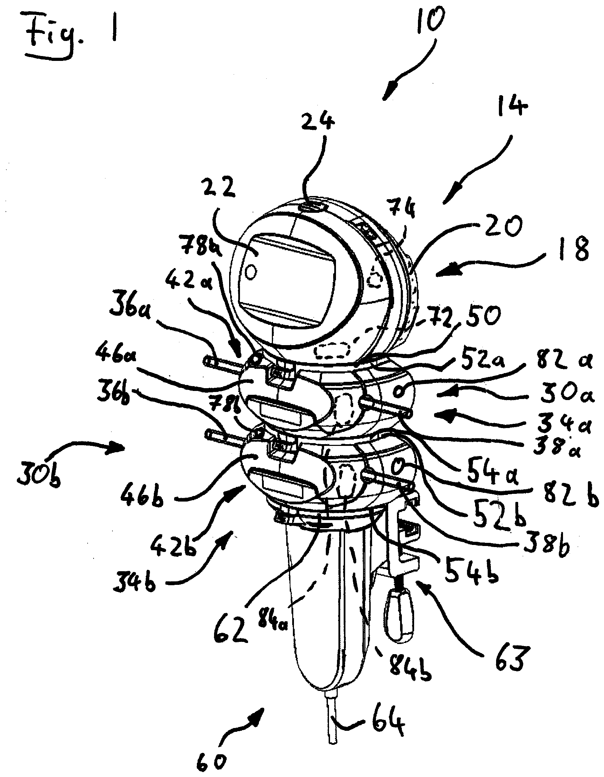

Figur 1 zeigt ein System 10 zur kombinierten Unterdruck und Instillationsbehandlung von Wunden am menschlichen oder tierischen Körper. Das System 10 umfasst eine Unterdruckeinheit 14 sowie beispielhaft zwei Instillationseinheiten 30a, 30b. Die Unterdruckeinheit 14 umfasst ein Gehäuse 18 einer ersten Gehäuseart. An dem Gehäuse 18 ist ein Flüssigkeitsaufnahmebehälter 20 lösbar befestigt. Dem Flüssigkeitsaufnahmebehälter 20 gegenüberliegend weist das Gehäuse 18 der ersten Gehäuseart eine Bedienoberfläche 22 auf. Die Bedienoberfläche 22 ist als berührungsempfindlicher Bildschirm ausgeführt. Der Flüssigkeitsaufnahmebehälter 20 ist mit dem Gehäuse 18 der ersten Gehäuseart beispielhaft über eine Schnappverbindung 24 verbunden. Durch Betätigung der Schnappverbindung 24 kann der Flüssigkeitsaufnahmebehälter 20 von dem Gehäuse 18 der ersten Gehäuseart gelöst werden.

Die Instillationseinheiten 30a, 30b umfassen je ein Gehäuse einer zweiten Gehäuseart. Sie weisen je einen eingehenden Leitungsabschnitt 36a, 36b sowie jeweils einen ausgehenden Leitungsabschnitt 38a, 38b auf. Sie umfassen jeweils einen Leitungsdurchführungsbereich 42a, 42b. Die Gehäuse 34a, 34b der zweiten Gehäuseart umfassen jeweils eine öffen- und verschließbare Abdeckung 46a, 46b. Über die jeweiligen Abdeckungen 46a, 46b sind die Leitungsdurchführungsbereiche 42a, 42b der Instillationseinheiten 30a, 30b zugänglich und werden vorzugsweise durch diese begrenzt.

Das Gehäuse 18 der ersten Art weist eine erste Befestigungseinrichtung 50 auf. Die Gehäuse 34a, 34b der zweiten Gehäuseart weisen jeweils eine zweite Befestigungseinrichtung 52a, 52b sowie jeweils eine dritte Befestigungseinrichtung 54a, 54b auf. Bezüglich der in Figur 1 dargestellten Betriebsposition des Systems 10 sind die ersten Befestigungseinrichtungen 52a, 52b jeweils an der Oberseite der Gehäuse 34a, 34b der zweiten Gehäuseart angebracht. Während die dritten Befestigungseinrichtungen 54a, 54b jeweils an den Unterseiten der Gehäuse 34a, 34b der zweiten Gehäuseart angebracht sind.

Die zweite, obere Befestigungseinrichtung 52a der ersten, oberen Instillationseinheit 30a ist dabei formschlüssig mit der ersten Befestigungseinrichtung 50 der Unterdruckeinheit 14 verbunden. Die zweite, obere Befestigungseinrichtung 52b der zweiten, unteren Instillationseinheit 30b ist formschlüssig mit der dritten, unteren Befestigungseinrichtung 54a der ersten, oberen Instillationseinheit 30a verbunden. Die dritte, untere Befestigungseinrichtung 54b der zweiten, unteren Instillationseinheit 30b ist über eine einer Haltevorrichtung 60 zugeordnete Befestigungseinrichtung 62 mit dieser Haltevorrichtung 60 verbunden. Über eine Klemmvorrichtung 63 können die miteinander verbundenen Einheiten 14, 30a, 30b beispielsweise an einem nicht gezeigten Infusionsständer oder einem Krankenbett befestigt werden.

In die Haltevorrichtung 60 geht eine Leitung 64 zur Spannungsversorgung ein. Im Bereich der Befestigungseinrichtungen 50, 52a, 52b, 54a, 54b, 62 sind jeweils Schnittstellen zur Spannungsversorgung vorgesehen, welche beim Verbinden der Einheiten 14, 30a, 30b und mit der Haltevorrichtung 60, miteinander in Kontakt treten.

Beispielhaft ist in der Unterdruckeinheit 14 eine Steuerungseinheit 72 des gesamten Systems angeordnet. Die Steuerungseinheit 72 ist mit einer Schnittstelle 74 verbunden, welche im vorliegenden Ausführungsbeispiel zur drahtlosen Kommunikation ausgelegt ist. Die Instillationseinheiten 30a, 30b weisen Schnittstellen 78a, 78b zur drahtlosen Kommunikation auf. Die Instillationseinheiten 30a, 30b weisen je einen Sensor 82a, 82b auf, auf welche später noch eingegangen wird. Die Instillationseinheiten 30a, 30b weisen je eine schematisch dargestellte Förderpumpe 84a, 84b auf, welche vorliegend als Peristaltikpumpen ausgeführt sind.

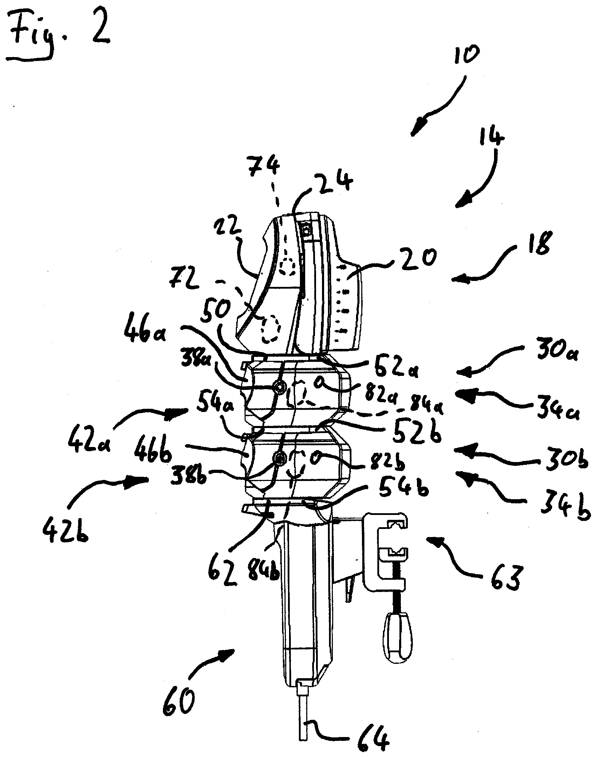

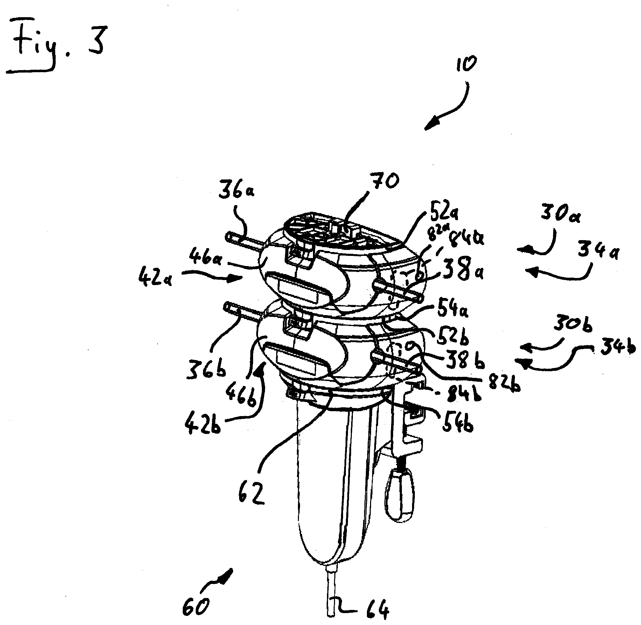

Figur 3 zeigt das erfindungsgemäße System aus Figur 1 und 2, jedoch ohne die Unterdruckeinheit 14. Die der oberen Instillationseinheit 30a zugeordnete Schnittstelle zur Spannungsversorgung ist in dieser Darstellung sichtbar und trägt das Bezugszeichen 70. Bei der Unterdruckeinheit 14 ist eine zu dieser Schnittstelle 70 komplementäre Schnittstelle zur Spannungsversorgung vorgesehen.

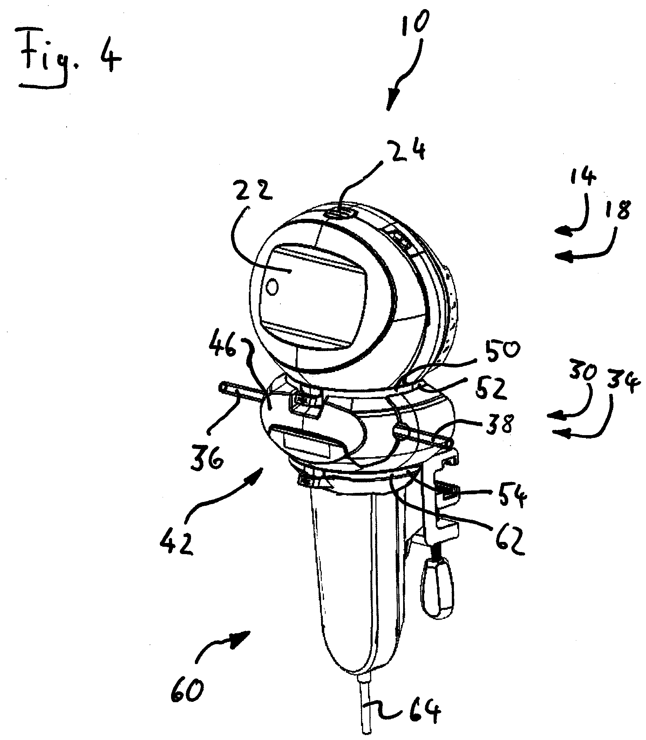

Figur 4 zeigt eine Ausführungsform des erfindungsgemäßen Systems 10 mit einer einzigen Instillationseinheit 30 und einer einzigen Unterdruckeinheit 14.

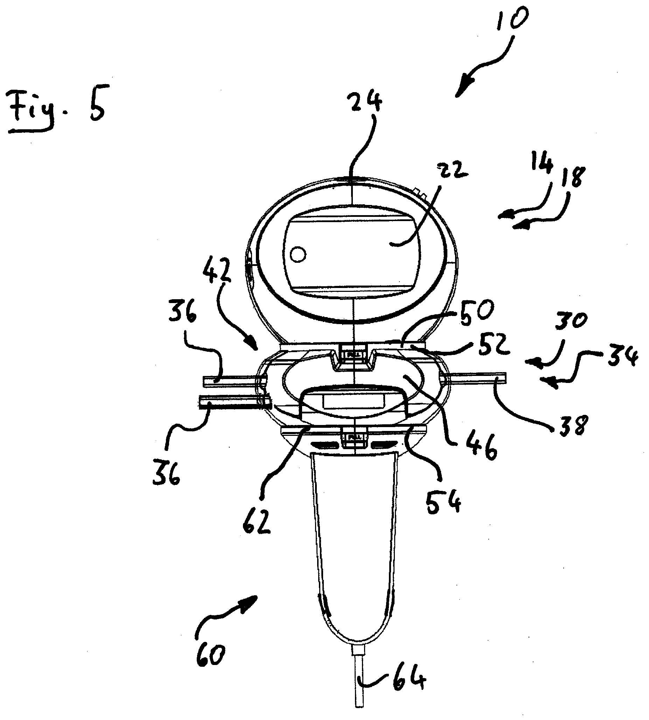

Die in Figur 5 gezeigte Ausführungsform unterscheidet sich von der in Figur 4 gezeigten Ausführungsform dadurch, dass die Instillationseinheit 30 zwei eingehende Leitungsabschnitte 36 und einen ausgehenden Leitungsabschnitt 38 aufweist. Im Leitungsdurchführungsbereich 42 sind die eingehenden Leitungsabschnitte 36 zusammengeführt und münden in den ausgehenden Leitungsabschnitt 54.

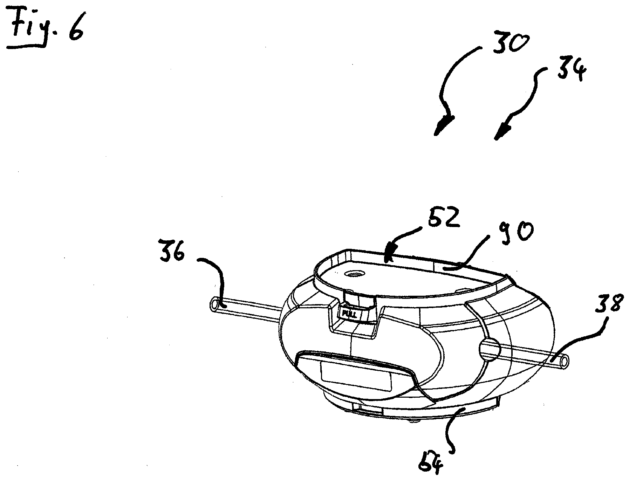

Figur 6 zeigt eine Instillationseinheit 30 mit beispielhaft einem eingehenden Leitungsabschnitt 36 und beispielhaft einem ausgehenden Leitungsabschnitt 38. Die zweite Befestigungseinrichtung 52 umfasst eine Umrandung 90. Diese Umrandung 90 ist ausgebildet, um beim Verbinden mit einer ersten Befestigungseinrichtung 50 oder einer dritten Befestigungseinrichtung 54 einen komplementär ausgebildeten Abschnitt der letzteren aufzunehmen.

Durch die Befestigungseinrichtungen 50, 52a, 52b, 54a, 54b, 62 sind die Instillationseinheit bzw. die Instillationseinheiten 30, 30a, 30b und die Unterdruckeinheit 14 der jeweiligen Systeme 10 miteinander verbindbar. Vorteilhafterweise sind dabei die Befestigungseinrichtungen 50, 52a, 52b, 54a, 54b, 62 derart ausgestaltet und angeordnet, dass die Instillationseinheiten 30, 30a, 30b und die Unterdruckeinheit 14 der Systeme 10 zu einem Turm zusammenfügbar sind, so wie dies in den Figuren 1, 2, 4 und 5 gezeigt ist.

Vorzugsweise ist in einer der Einheiten 14, 30, 30a, 30b eine Master-Steuerungseinheit 72 vorgesehen, die den Betrieb aller Einheiten steuert. Die Steuerungseinheit 72 bestimmt dabei die Betriebsweise der Unterdruckeinheit 14 und der Instillationseinheiten 30, 30a, 30b. In der in den Figuren 1 und 2 gezeigten Ausführungsform übermittelt die Steuerungseinheit 72 Daten bezüglich der Betriebsweise über die der Unterdruckeinheit 14 zugeordnete Schnittstelle 74 an die Schnittstellen 78a, 78b der Instillationseinheiten 30a, 30b.

Die bei den Instillationseinheiten 30a, 30b vorgesehenen Sensoren 82a, 82b detektieren, wenn sich in den eingehenden Leitungsabschnitten 36a, 36b keine Instillationsflüssigkeit befindet. Ist dies der Fall, so kann der Betrieb der jeweiligen Peristaltikpumpe 84a, 84b eingestellt werden und ein Signal ausgegeben werden, welches zum Bereitstellen weiterer Instillationsflüssigkeit auffordert.

## Patentansprüche

1. System (10) zur kombinierten Unterdruck- und Instillationsbehandlung von Wunden am menschlichen oder tierischen Körper, wobei das System (10) umfasst, eine Unterdruckeinheit (14) mit einem Gehäuse (18) einer ersten Gehäuseart, mit einer Pumpe zur Bereitstellung von Unterdruck und mit einem Flüssigkeitsaufnahmebehälter (20) zur Aufnahme von Körperflüssigkeiten, insbesondere von aus einer Wunde abgesaugten Wundsekreten, wobei der Flüssigkeitsaufnahmebehälter (20) lösbar an dem Gehäuse (18) der ersten Gehäuseart befestigbar ist, und eine Instillationseinheit (30, 30a, 30b) mit einem Gehäuse (34, 34a, 34b) einer zweiten Gehäuseart, mit einem Leitungsdurchführungsbereich (42) für mindestens einen eingehenden Leitungsabschnitt (36, 36a, 36b) und einen ausgehenden Leitungsabschnitt (38, 38a, 38b), mit einer Förderpumpe (84a, 84b), vorzugsweise einer Peristaltikpumpe, wobei die Gehäuse (18, 34, 34a, 34b) der ersten und der zweiten Gehäuseart jeweils separate voneinander getrennte oder voneinander trennbare Gehäuse (18, 34, 34a, 34b) sind, **dadurch gekennzeichnet, dass** das Gehäuse (34, 34a, 34b) der zweiten Gehäuseart eine öffenbare und verschließbare Abdeckung (46, 46a, 46b) aufweist, über die der Leitungsdurchführungsbereich (42) zugänglich ist, und dass das Gehäuse (18) der ersten Gehäuseart eine erste Befestigungseinrichtung (50) aufweist und dass das Gehäuse (34, 34a, 34b) der zweiten Gehäuseart eine zweite Befestigungseinrichtung (52, 52a, 52b) aufweist, wobei die erste Befestigungseinrichtung (50) mit der zweiten Befestigungseinrichtung (52, 52a, 52b) formschlüssig, vorzugsweise schnappend, rastend oder in sonstiger Weise formschlüssig hintergreifend, oder klemmend verbindbar ist.

2. System nach Anspruch 1, **dadurch gekennzeichnet, dass** die Abdeckung (46, 46a, 46b) schwenkbar an dem Gehäuse der zweiten Gehäuseart (34, 34a, 34b) angebracht ist.

3. System nach einem der vorangegangenen Ansprüche 1 oder 2, **dadurch gekennzeichnet, dass** bezüglich einer Betriebsposition des Systems (10) die Abdeckung (46, 46a, 46b) seitlich oder hinten oder vorn an dem Gehäuse (34, 34a, 34b) der zweiten Gehäuseart angebracht ist.

4. System nach einem der vorangegangenen Ansprüche, **dadurch gekennzeichnet, dass** die Abdeckung (46, 46a, 46b) den Leitungsdurchführungsbereich (42) der Instillationseinheit (30, 30a, 30b) begrenzt.

5. System nach einem der vorangegangenen Ansprüche, **dadurch gekennzeichnet, dass** bezüglich einer Betriebsposition des Systems (10) die erste Befestigungseinrichtung (50) an einer Unterseite des Gehäuses (18) der ersten Gehäuseart angeordnet ist und die zweite Befestigungseinrichtung (52, 52a, 52b) an einer Oberseite des Gehäuses (34, 34a, 34b) der zweiten Gehäuseart angeordnet ist.

6. System nach einem der vorangegangenen Ansprüche, **dadurch gekennzeichnet, dass** das Gehäuse (34, 34a, 34b) der zweiten Gehäuseart mindestens eine dritte Befestigungseinrichtung (54, 54a, 54b) aufweist, wobei diese dritte Befestigungseinrichtung (54, 54a, 54b) mit einer zweiten Befestigungseinrichtung (52, 52a, 52b) eines weiteren Gehäuses (34, 34a, 34b) der zweiten Gehäuseart formschlüssig, vorzugsweise schnappend, rastend oder in sonstiger Weise formschlüssig hintergreifend, oder klemmend verbindbar ist und dass die dritte Befestigungseinrichtung (54, 54a, 54b) bezüglich einer Betriebsposition des Systems vorzugsweise an einer Unterseite des Gehäuses (34, 34a, 34b) der zweiten Gehäuseart angeordnet ist.

7. System nach einem der vorangegangenen Ansprüche, **dadurch gekennzeichnet, dass** es eine Haltevorrichtung (60) mit einer der Haltevorrichtung (60) zugeordneten Befestigungseinrichtung (62) umfasst, wobei diese der Haltevorrichtung (60) zugeordnete Befestigungseinrichtung (62) mit der ersten Befestigungseinrichtung (50) des Gehäuses (18) der ersten Gehäuseart und/oder der dritten Befestigungseinrichtung (54, 54a, 54b) des Gehäuses (34, 34a, 34b) der zweiten Gehäuseart formschlüssig, vorzugsweise schnappend, rastend oder in sonstiger Weise formschlüssig hintergreifend, oder klemmend verbindbar ist.

8. System nach Anspruch 7, **dadurch gekennzeichnet, dass** die Instillationseinheit (30, 30a, 30b) eine Schnittstelle zur Spannungsversorgung aufweist, welche mit einer komplementären Schnittstelle zur Spannungsversorgung bei der Haltevorrichtung (60) verbindbar ist.

9. System nach einem der vorangegangenen Ansprüche, **dadurch gekennzeichnet, dass** die Instillationseinheit (30, 30a, 30b) eine Schnittstelle (70) zur Spannungsversorgung aufweist, welche mit einer komplementären Schnittstelle zur Spannungsversorgung der Unterdruckeinheit (14) verbindbar ist.

10. System nach einem der vorangegangenen Ansprüche, **dadurch gekennzeichnet, dass** der Leitungsdurchführungsbereich (42, 42a, 42b) für mindestens zwei eingehende Leitungsabschnitte (36, 36a, 36b) und mindestens einen ausgehenden Leitungsabschnitt (38, 38a, 38b) ausgelegt ist.

11. System nach Anspruch 10, **dadurch gekennzeichnet, dass** die eingehenden Leitungsabschnitte (36, 36a, 36b) in dem Leitungsdurchführungsbereich (42, 42a, 42b) zusammengeführt sind und in den ausgehenden Leitungsabschnitt (38, 38a, 38b) münden.

12. System nach Anspruch 10 oder 11, **dadurch gekennzeichnet, dass** die Förderpumpe (84a, 84b) derart im Bereich des ausgehenden Leitungsabschnitts (38, 38a, 38b) angeordnet und ausgebildet ist, dass sie im Betrieb fluidfördernd auf den ausgehenden Leitungsabschnitts (38, 38a, 38b) einwirkt.

13. System nach einem der vorangegangenen Ansprüche, **dadurch gekennzeichnet, dass** die Instillationseinheit (30, 30a, 30b) einen Sensor (82a, 82b) aufweist, der entlang einer Fluidförderrichtung vor der Förderpumpe (84a, 84b) angeordnet ist und ausgebildet ist, um zu detektieren, wenn der eingehende Leitungsabschnitt (36, 36a, 36b) keine Instillationsflüssigkeit enthält.

14. System nach einem der vorangegangenen Ansprüche, **dadurch gekennzeichnet, dass** die Abdeckung (46, 46a, 46b) ausgebildet ist um im geschlossenen Zustand den Leitungsdurchführungsbereich (42, 42a, 42b) zu begrenzen und einen Leitungsabschnitt zu führen und/oder zu fixieren.

15. System nach einem der vorangegangenen Ansprüche, **dadurch gekennzeichnet, dass** der Leitungsdurchführungsbereich (42, 42a, 42b) mindestens ein Mittel aufweist, das angeordnet und ausgelegt ist, um einen im Leitungsdurchführungsbereich (42, 42a, 42b) anordenbaren Leitungsabschnitt fluidisch zu unterbrechen, insbesondere abzuklemmen.

## Claims

1. A system (10) for combined vacuum and instillation treatment of wounds on the human or animal body, wherein the system (10) comprises a vacuum unit (14) with a housing (18) of a first housing type, with a pump for providing a vacuum, and with a liquid collection container (20) for receiving bodily fluids, in particular wound secretions aspirated from a wound, wherein the liquid collection container (20) can be secured releasably on the housing (18) of the first housing type, and an instillation unit (30, 30a, 30b) with a housing (34, 34a, 34b) of a second housing type, with a conduit passage area (42) for at least one inward conduit portion (36, 36a, 36b) and an outward conduit portion (38, 38a, 38b), with a feed pump (84a, 84b), preferably a peristaltic pump, wherein the housings (18, 34, 34a, 34b) of the first and second housing types are each housings (18, 34, 34a, 34b) that are separate from each other or separable from each other, **characterized in that** the housing (34, 34a, 34b) of the second housing type has an openable and closable cover (46, 46a, 46b) through which the conduit passage area (42) is accessible, and **in that** the housing (18) of the first housing type has a first securing mechanism (50), and **in that** the housing (34, 34a, 34b) of the second housing type has a second securing mechanism (52, 52a, 52b), wherein the first securing mechanism (50) can be connected to the second securing mechanism (52, 52a, 52b) with a form fit, preferably a snap fit, a latching fit, or some other form-fit engagement from behind, or a clamping fit.

2. The system as claimed in claim 1, **characterized in that** the cover (46, 46a, 46b) is mounted pivotably on the housing of the second housing type (34, 34a, 34b).

3. The system as claimed in either of claims 1 and 2, **characterized in that**, with respect to an operating position of the system (10), the cover (46, 46a, 46b) is mounted on the side or rear or front of the housing (34, 34a, 34b) of the second housing type.

4. The system as claimed in one of the preceding claims, **characterized in that** the cover (46, 46a, 46b) delimits the conduit passage area (42) of the instillation unit (30, 30a, 30b).

5. The system as claimed in one of the preceding claims, **characterized in that**, with respect to an operating position of the system (10), the first securing mechanism (50) is arranged on an underside of the housing (18) of the first housing type, and the second securing mechanism (52, 52a, 52b) is arranged on a top of the housing (34, 34a, 34b) of the second housing type.

6. The system as claimed in one of the preceding claims, **characterized in that** the housing (34, 34a, 34b) of the second housing type has at least a third securing mechanism (54, 54a, 54b), wherein this third securing mechanism (54, 54a, 54b) can be connected to a second securing mechanism (52, 52a, 52b) of a further housing (34, 34a, 34b) of the second housing type with a form fit, preferably a snap fit, a latching fit, or some other form-fit engagement from behind, or a clamping fit, and **in that** the third securing mechanism (54, 54a, 54b), with respect to an operating position of the system, is preferably arranged on an underside of the housing (34, 34a, 34b) of the second housing type.

7. The system as claimed in one of the preceding claims, **characterized in that** it comprises a holding device (60), with a securing mechanism (62) assigned to the holding device (60), wherein this securing mechanism (62) assigned to the holding device (60) can be connected to the first securing mechanism (50) of the housing (18) of the first housing type and/or to the third securing mechanism (54, 54a, 54b) of the housing (34, 34a, 34b) of the second housing type with a form fit, preferably a snap fit, a latching fit, or some other form-fit engagement from behind, or a clamping fit.

8. The system as claimed in claim 7, **characterized in that** the instillation unit (30, 30a, 30b) has a voltage supply interface, which can be connected to a complementary voltage supply interface in the holding device (60).

9. The system as claimed in one of the preceding claims, **characterized in that** the instillation unit (30, 30a, 30b) has a voltage supply interface (70), which can be connected to a complementary voltage supply interface in the vacuum unit (14).

10. The system as claimed in one of the preceding claims, **characterized in that** the conduit passage area (42, 42a, 42b) is designed for at least two inward conduit portions (36, 36a, 36b) and at least one outward conduit portion (38, 38a, 38b).

11. The system as claimed in claim 10, **characterized in that** the inward conduit portions (36, 36a, 36b) are brought together in the conduit passage area (42, 42a, 42b) and open into the outward conduit portion (38, 38a, 38b).

12. The system as claimed in claim 10 or 11, **characterized in that** the feed pump (84a, 84b) is arranged and designed in the area of the outward conduit portion (38, 38a, 38b) in such a way that, during operation, it exerts a fluid-conveying action on the outward conduit portion (38, 38a, 38b).

13. The system as claimed in one of the preceding claims, **characterized in that** the instillation unit (30, 30a, 30b) has a sensor (82a, 82b) which is arranged upstream from the feed pump (84a, 84b) along a fluid delivery direction and is designed to detect when the inward conduit portion (36, 36a, 36b) contains no instillation liquid.

14. The system as claimed in one of the preceding claims, **characterized in that** the cover (46, 46a, 46b) is designed such that, in the closed state, it delimits the conduit passage area (42, 42a, 42b) and guides and/or fixes a conduit portion.

15. The system as claimed in one of the preceding claims, **characterized in that** the conduit passage area (42, 42a, 42b) has at least one means which is arranged and designed to fluidically interrupt, in particular to clamp shut, a conduit portion that can be arranged in the conduit passage area (42, 42a, 42b).

## Revendications

1. Système (10) de traitement combiné de plaies, par dépression et instillation, sur le corps humain ou animal, le système (10) comprenant une unité de dépression (14) comportant un boîtier (18) d'un premier type de boîtier, une pompe servant à produire une dépression et un récipient de réception de liquides (20) destiné à recevoir des liquides corporels, en particulier des sécrétions aspirées au niveau d'une plaie, dans lequel le récipient de réception de liquides (20) peut être fixé de manière amovible au boîtier (18) du premier type de boîtier, et une unité d'instillation (30, 30a, 30b) comportant un boîtier (34, 34a, 34b) d'un deuxième type de boîtier, une zone de passage de conduit (42) destinée à au moins une portion de conduit entrant (36, 36a, 36b) et une portion de conduit sortant (38, 38a, 38b), une pompe d'alimentation (84a, 84b), de préférence une pompe péristaltique, dans lequel les boîtiers (18, 34, 34a, 34b) des premier et deuxième types de boîtier sont chacun des boîtiers distincts (18, 34, 34a, 34b) qui sont séparés ou peuvent être séparés l'un de l'autre, **caractérisé par le fait que** le boîtier (34, 34a, 34b) du deuxième type de boîtier comprend une couverture (46, 46a, 46b) apte à être ouverte et fermée qui permet d'accéder à la zone de passage de conduit (42), et **par le fait que** le boîtier (18) du premier type de boîtier comprend un premier dispositif de fixation (50), et **par le fait que** le boîtier (34, 34a, 34b) du deuxième type de boîtier comprend un deuxième dispositif de fixation (52, 52a, 52b), le premier dispositif de fixation (50) pouvant être relié à engagement positif, de préférence par enclenchement, par encliquetage ou d'une autre façon à engagement positif par prise de derrière, ou par serrage, au deuxième dispositif de fixation (52, 52a, 52b).

2. Système selon la revendication 1, **caractérisé par le fait que** ladite couverture (46, 46a, 46b) est montée à pivotement sur le boîtier du deuxième type de boîtier (34, 34a, 34b).

3. Système selon l'une quelconque des revendications précédentes 1 ou 2, **caractérisé par le fait que**, par rapport à une position de service du système (10), la couverture (46, 46a, 46b) est montée latéralement ou à l'arrière ou à l'avant sur le boîtier (34, 34a, 34b) du deuxième type de boîtier.

4. Système selon l'une quelconque des revendications précédentes, **caractérisé par le fait que** la couverture (46, 46a, 46b) délimite la zone de passage de conduit (42) de l'unité d'instillation (30, 30a, 30b).

5. Système selon l'une quelconque des revendications précédentes, **caractérisé par le fait que**, par rapport à une position de service du système (10), le premier dispositif de fixation (50) est disposé sur une face inférieure du boîtier (18) du premier type de boîtier et le deuxième dispositif de fixation (52, 52a, 52b) est disposé sur une face supérieure du boîtier (34, 34a, 34b) du deuxième type de boîtier.

6. Système selon l'une quelconque des revendications précédentes, **caractérisé par le fait que** le boîtier (34, 34a, 34b) du deuxième type de boîtier comprend au moins un troisième dispositif de fixation (54, 54a, 54b), dans lequel ce troisième dispositif de fixation (54, 54a, 54b) peut être relié à engagement positif, de préférence par enclenchement, par encliquetage ou d'une autre façon à engagement positif par prise de derrière, ou par serrage, à un deuxième dispositif de fixation (52, 52a, 52b) d'un autre boîtier (34, 34a, 34b) du deuxième type de boîtier.

7. Système selon l'une quelconque des revendications précédentes, **caractérisé par le fait qu'**il comprend un dispositif de maintien (60) ayant un dispositif de fixation (62) associé au dispositif de maintien (60), dans lequel ce dispositif de fixation (62) associé au dispositif de maintien (60) peut être relié à engagement positif, de préférence par enclenchement, par encliquetage ou d'une autre façon à engagement positif par prise de derrière, ou par serrage, au premier dispositif de fixation (50) du boîtier (18) du premier type de boitier et/ou au troisième dispositif de fixation (54, 54a, 54b) du boîtier (34, 34a, 34b) du deuxième type de boîtier.

8. Système selon la revendication 7, **caractérisé par le fait que** ladite unité d'instillation (30, 30a, 30b) présente une interface d'alimentation en tension qui peut être reliée à une interface complémentaire d'alimentation en tension dans le dispositif de maintien (60).

9. Système selon l'une quelconque des revendications précédentes, **caractérisé par le fait que** ladite unité d'instillation (30, 30a, 30b) présente une interface (70) d'alimentation en tension qui peut être reliée à une interface complémentaire d'alimentation en tension de l'unité de dépression (14).

10. Système selon l'une quelconque des revendications précédentes, **caractérisé par le fait que** la zone de passage de conduit (42, 42a, 42b) est conçue pour au moins deux portions de conduit entrant (36, 36a, 36b) et au moins une portion de conduit sortant (38, 38a, 38b).

11. Système selon la revendication 10, **caractérisé par le fait que** les portions de conduit entrant (36, 36a, 36b) sont réunies dans la zone de passage de conduit (42, 42a, 42b) et débouchent dans la portion de conduit sortant (38, 38a, 38b).

12. Système selon la revendication 10 ou 11, **caractérisé par le fait que** la pompe d'alimentation (84a, 84b) est disposée et réalisée au niveau de la portion de conduit sortant (38, 38a, 38b) de telle manière que, en service, elle agit à transport de fluide sur la portion de conduit sortant (38, 38a, 38b).

13. Système selon l'une quelconque des revendications précédentes, **caractérisé par le fait que** ladite unité d'instillation (30, 30a, 30b) comprend un capteur (82a, 82b) qui est disposé en amont de la pompe d'alimentation (84a, 84b) suivant une direction de transport de fluide et est configuré pour détecter si la portion de conduit entrant (36, 36a, 36b) ne contient pas de liquide d'instillation.

14. Système selon l'une quelconque des revendications précédentes, **caractérisé par le fait que** ladite couverture (46, 46a, 46b) est configurée pour délimiter en état fermé la zone de passage de conduit (42, 42a, 42b) et pour guider et/ou fixer une portion de conduit.

15. Système selon l'une quelconque des revendications précédentes, **caractérisé par le fait que** la zone de passage de conduit (42, 42a, 42b) présente au moins un moyen qui est disposé et agencé pour interrompre fluidiquement, en particulier débrancher, une portion de conduit apte à être disposée dans la zone de passage de conduit (42, 42a, 42b).
